# EUROPEAN PATENT APPLICATION

(11) **EP 2 233 172 A1**
(43) Date of publication of application: **29.09.2010**
(21) Application number: 08839949.8
(22) Date of filing: 15.10.2008
(51) Int. Cl.: A61N 1/36

(54) **BLOOD PRESSURE STABILIZATION SYSTEM USING TRANSDERMAL STIMULATION**

(30) Priority: 15.10.2007 US 998979 P
(71) Applicant: Kyushu University, National University Corporation, Higashi-ku Fukuoka-shi Fukuoka 812-8581 (JP)
(72) Inventor: SUNAGAWA, Kenji, Fukuoka-shi Fukuoka 812-8581 (JP); CHISHAKI, Akiko, Fukuoka-shi Fukuoka 812-8581 (JP); YOSHIDA, Masayoshi, Fukuoka-shi Fukuoka 812-8581 (JP)
(74) Representative: Dunleavy, Kevin James
(86) International application number: PCT/JP2008/002922
(87) International publication number: WO 2009/050887

(57) **Abstract**

The present invention relates to an electric stimulation apparatus for treating hypotension of patients with spinal cord injury and a method for treating hypotension. An electric stimulation apparatus of the present invention comprises: a blood pressure measuring means for continuously measuring a blood pressure of a subject; an electric current application means for intermittently applying an electric current to skin of the subject; and a control means for controlling the electric current application means so as to maintain the blood pressure at a predetermined target blood pressure value by activating the electric current application means when the subject blood pressure is equal to or less than the target blood pressure value.

## Description

### FIELD OF THE INVENTION

This application claims priority under EPC, Article 87 based upon Provisional Patent Application No. 60/998,979, filed on October 15, 2007. The entire disclosures of the aforesaid applications are incorporated herein by reference.

The present invention relates to an electric stimulation apparatus for treating hypotension and a method for treating hypotension, and more specifically relates to an electric stimulation apparatus and a method used for treating patients with spinal cord injury.

### BACKGROUND OF THE INVENTION

Patients with spinal cord injury are known to have hypotension due to autonomic dysreflexia caused by the damage to the spinal cord, which is an autonomic nerve pathway. The spinal cord is an important nerve pathway and its failure due to, for example, a trauma blocks the neural transmission channels, resulting in motor paralysis, sensory paralysis, autonomic disorder or the like in downstream from the injury site. In Japan, about 5,000 people suffer spinal cord injuries annually and most of them occur in the higher level, thus 68% of these patients, 35% of which require vasopressor. develop hypotension

For patients with spinal cord injury, hypotension is a serious condition. Particularly orthostatic hypotension causes a sudden fall in blood pressure when the patient stands up, or sits up from the supine position, resulting in dizziness, lightheadedness or in severe cases unconsciousness. In order to control hypotension, various drug treatments have been performed, but with great difficulties since this type of hypotension is caused by the autonomic dysreflexia.

On the other hand, it is empirically known that mechanical or electrical stimulation increases the blood pressure, but this phenomenon has not been taken advantage of in the hypotension treatment application.

Although there is a method for directly and electrically stimulating the spinal cord in order to prevent the blood pressure decrease during a surgery, this method requires an invasive procedure for positioning a catheter near the spinal cord by a surgical technique, adding undesirable burden to the patient's body. In addition, this method requires to keep the invasive catheter attached to the patient on the daily basis, compromising the quality of life (QOL) of the patient with spinal cord injury.

### SUMMARY OF THE INVENTION

Considering the above situation, the purpose of the present invention is to provide an apparatus capable of preventing and treating orthostatic hypotension with electric stimulation using a non-invasive method.

In order to address the aforementioned problems, according to a first principal aspect of the present invention, there is provided an electric stimulation apparatus for treating hypotension, comprising: a blood pressure measuring means for continuously measuring a blood pressure of a subject; an electric current application means for intermittently applying an electric current with a predetermined frequency to skin of the subject to thereby stimulate the subject skin; and a control means for controlling the electric current application means so as to maintain the blood pressure at a predetermined target blood pressure value by activating the electric current application means to stimulate the subject skin when the subject blood pressure is equal to or less than the target blood pressure value.

According to such a structure, a decrease in blood pressure may be prevented using a non-invasive method in which an electric current is applied to the patient skin. In other words, the present inventors found that the electric stimulation applied to the skin may provide a stable blood pressure increase response. Also, since these response characteristics are generally consistent for any patient, an apparatus can be made with a relatively simple structure capable of stabilizing the patient blood pressure using, for example, a feedback control.

According to one embodiment of the present invention, the electric current intermittently applied by the electric current application means has an application period shorter than a non-application period, and it is preferred that the application period is 1-3 seconds and the non-application period is 4-10 seconds.

By applying such an intermittent electric current, stable effect of blood pressure increase may be provided continuously over an extended period.

According to another embodiment of the present invention, the electric current application means comprises an electrode positioned within inguinal, femoral, lumbar and lower abdominal regions of the subject.

According to such a structure, a maximum effect may be achieved with minimum electric power or amperage.

According to yet another embodiment, the control means maintains the blood pressure of the subject at the target blood pressure value with a feedback control. In this case, the control means preferably controls the applied electric current at a predetermined frequency.

According to still another embodiment, this apparatus preferably further comprises an operation means for entering the target blood pressure value. In addition, the electric stimulation apparatus is preferably secured to a wheelchair.

According to a second principal aspect of the present invention, there is provided an electric stimulation method for treating hypotension, comprising the steps of: continuously measuring a blood pressure of a subject; intermittently applying an electric current with a predetermined frequency to skin of the subject to thereby stimulate the subject skin; and controlling an electric current application means so as to maintain the blood pressure at a predetermined target blood pressure value by activating the electric current application means to stimulate the subject skin when the subject blood pressure is equal to or less than the target blood pressure value.

The above and other characteristics of the present invention will be readily appreciated by those skilled in the art by referring to the following Detailed Description of the Preferred Embodiments and the accompanying drawings.

### BRIEF DESCRIPTION OF THE DRAWINGS

Fig. 1 is a schematic structural view showing an electric stimulation apparatus of the present invention;

Fig. 2 is a graph showing changes in (a) heart rate and (b) blood pressure measured when (c) bed inclination angle is changed for a patient with orthostatic hypotension in the supine position;

Fig. 3A is a graph showing the systolic blood pressure change for patients with severe hypotension due to cervical and thoracic spinal cord injuries (10 patients with various injury levels in C4A-C5A), wherein the each patient in the supine position on a tilt table was raised to a 30 degree and to a 60 degree angle;

Fig. 3B is a graph showing the mean blood pressure change for patients with severe hypotension due to cervical and thoracic spinal cord injuries (10 patients with various injury levels in C4A-C5A), wherein the each patient in the supine position on a tilt table was raised to a 30 degree and to a 60 degree angle;

Fig. 4A is a graph showing the observed blood pressure change due to the stimulus applied to a patient, wherein each of the inguinal, femoral, lower leg, near umbilicus and subclavian regions of the patient was stimulated by pinching;

Fig. 4B is a graph showing the observed systolic blood pressure change due to the stimulus applied to a patient, wherein each of the lower leg, knee, femoral, inguinal, abdominal and subclavian regions of four patients was stimulated by pinching;

Fig. 5A is a diagram showing the heart rate and blood pressure change measured when intermittent electric stimulation was applied within the lumbar (1) and lower leg (6) regions of the patient, where in the stimulation had settings of 40 mA, 40 Hz, elctrode area 8 cm² and 0.3 msec and intervals of 2 sec. ON and 6 sec. OFF;

Fig. 5B is a diagram showing the mean blood pressure increase measured when electric stimulation of 5 mA/cm², 10 Hz, 0.3 msec, elctrode area 8 cm² was applied to each of the lower leg, femoral, inguinal and lower abdominal regions of patients;

Fig. 6A is a graph showing the blood pressure change observed continuously when attaching an electrode 4 to the patient inguinal region; and applying the electric current with a constant frequency at 10 Hz, square wave stimulations of 0.3 msec interval and different amperage values 10 mA, 20mA, 40mA and 60mA, elctrode area 8 cm²;

Fig. 6B is a graph showing the changes in systolic blood pressure (SBP), mean blood pressure (MBP) and diastolic blood pressure (DBP) measured when an electrode 4 was attached to the patient inguinal region; and an electric current was applied with a constant frequency at 10 Hz, square wave stimulations of 0.3 msec interval and different current values 1.3 mA/cm², 2.5 mA/cm², 5 mA/cm² and 7.5mA/cm^{2,} elctrode area 8 cm²;

Fig. 7A is a graph showing the blood pressure change measured when an electric current was applied with a constant amperage 40 mA, square wave stimulations of 0.3 msec interval and varying the frequency from 0 Hz to 10 Hz, elctrode area 8 cm²;

Fig. 7B is a graph showing the changes in systolic blood pressure (SBP), mean blood pressure (MBP) and diastolic blood pressure (DBP) measured when an electric current was applied with a constant amperage 40 mA, square wave stimulations of 0.3 msec interval and different frequencies of 5 Hz, 10 Hz, 20 Hz and 40 Hz, elctrode area 8 cm²;

Fig. 8 is a graph showing the blood pressure increase in response to the electric current applied to a patient with various intermittent time intervals (continuous; 2 sec. ON and 2 sec. OFF; 2 sec. ON and 4 sec. OFF; 2 sec. ON and 6 sec. OFF; and 2 sec. and 10 sec. OFF, elctrode area 8 cm²);

Fig. 9A is a diagram showing electric current-blood pressure dynamic characteristics measured when an electric current with a constant frequency was applied to patients;

Fig. 9B is a diagram showing electric current-blood pressure dynamic characteristics measured when an electric current with a constant frequency was applied to patients;

Fig. 10 are a block schematic diagram for designing a feedback-based controller (a); and a graph showing the response characteristics for different sets of the coefficients Kp and Ki (b);

Fig. 11 shows the results of the blood pressure change in an actual study patient, wherein the varying electric stimulation was applied to the patient under the condition that the maximum amperage was 60 mA, target blood pressure value 85mmHg, frequency 10 Hz, stimulation interval 0.3 msec, 2 sec. on / 6 sec. off, and electrode area 8 cm²;

Fig. 12 is a graph showing the blood pressure change measured when the target blood pressure value was set to 85 mmHg, the frequency was kept constant and the electric current (amperage) was controlled;

Fig. 13 is a graph showing the blood pressure change measured when the target blood pressure value was set to 65 mmHg, the electric current (amperage) was kept constant and the frequency was controlled; and

Fig. 14 is a diagram showing the average blood pressure change for 7 cases when the frequency was kept constant at 10 Hz, the maximum electric current (amperage) was set to 60 mA, and the current was controlled.

### DETAILED DESCRIPTION OF THE INVENTION

One preferred embodiment of the present invention will be described in detail below.

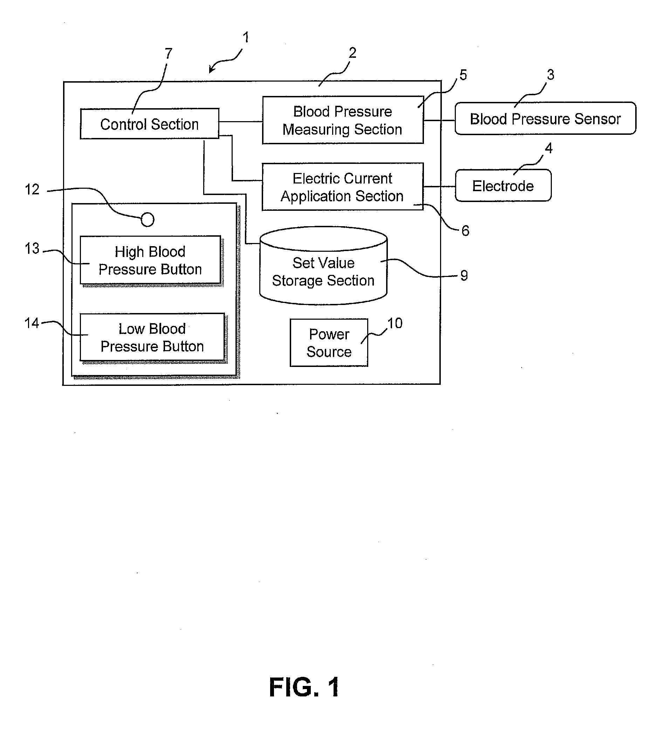

Fig. 1 is a schematic structural view illustrating an electric stimulation apparatus 1 according to this embodiment.

This apparatus 1 is defined by an apparatus main body 2 connected to a blood pressure sensor 3 and an electrode 4. The apparatus main body 2 is provided with a blood pressure measuring section 5, an electric current application section 6, a control section 7, an interface 8, a set value storage section 9 and a power source 10.

The electric current application section 6 is connected to the electrode 4 and has a function to intermittently apply an electric current with a predetermined frequency to human skin through the electrode 4. The electrode 4 has an area of about 5 cm² to 10 cm² for applying the effective electric current and is preferably placed on the patient skin within the inguinal, femoral, lumbar and lower abdominal regions.

Also the blood pressure measuring section 5 has a function to continuously measure the patient blood pressure with the blood pressure sensor 3. This blood pressure sensor 3 may be an existing one and may be a non-invasive sensor, for example, an optical sensor or a wired/wireless blood pressure sensor using techniques such as the pulse wave velocity measurement method.

The control section 7 has a function to activate the electric current application section 6 to stimulate the patient skin when the patient blood pressure is equal to or less than a predetermined target blood pressure value, and control the electric current application section 6 so as to maintain the blood pressure at (or greater than) the target blood pressure value. This control section 7 is adapted to dynamically control the electric current applied to the electrode 4 using a feedback control as discussed below.

The interface 8 is for setting the target blood pressure value and has, for example, a pilot lamp (indicator light) 11, a high blood pressure button 12 and a low blood pressure button 13 so as to configure a standard target blood pressure value and other target blood pressure values corresponding to each of these buttons, as shown in Fig. 1. In addition, this operation interface 8 may be provided with a digital blood pressure display section (not shown) for displaying the target blood pressure value and/or measurements or, for example, a up and down buttons for performing fine adjustments.

The set value storage section 9 stores the target blood pressure value configured at the operation interface 8; an amperage, a frequency and other operational characteristics of the electrode 4; measurement characteristics of the blood pressure sensor 3; and other settings. These settings are adapted to be retrieval by the control section 7 and used for the feedback control. This set value storage section 9 also stores a time interval for activating the electrode 4 (or electric current application section 6). As will be described below, the electrode activation is intermittent and the time intervals are preferably configured so that the current is applied for a range of 1-3 seconds and stopped for a range of 4-10 seconds.

Configuration and operation of this apparatus and an exemplary method for treating hypotension using this apparatus will be described in detail below.

Blood Pressure Change in Patients with Cervical Spinal Cord Injury

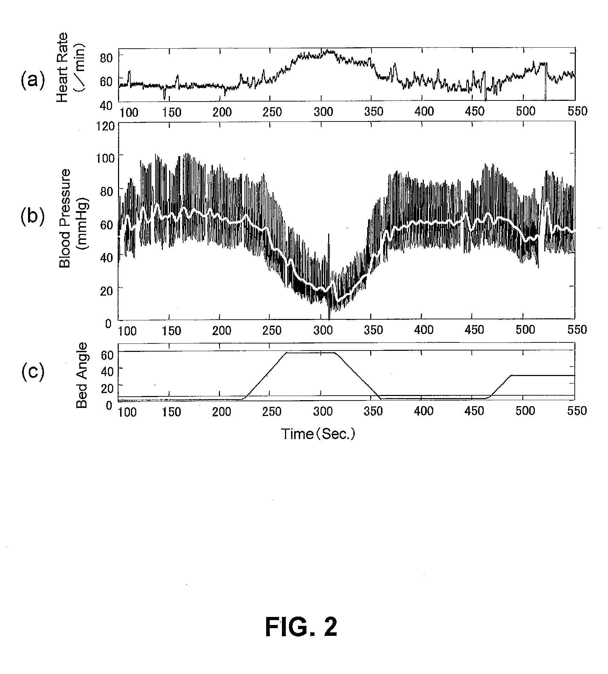

Fig. 2 is a graph describing orthostatic hypotension treated with the apparatus according to the present embodiment. In this figure, (a) is the patient heart rate; (b) is the patient blood pressure; and (c) is bed inclination angle for a patient with severe hypotension. As shown in (b), the blood pressure decreases significantly as the bed inclination angle increases from 0 degree to 60 degree.

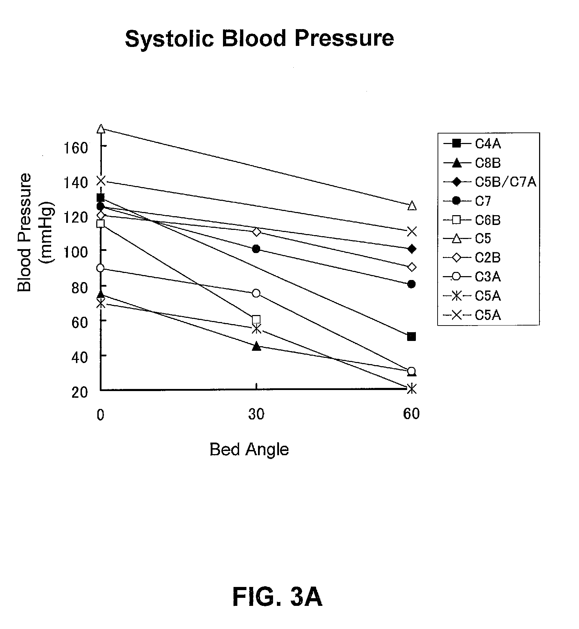

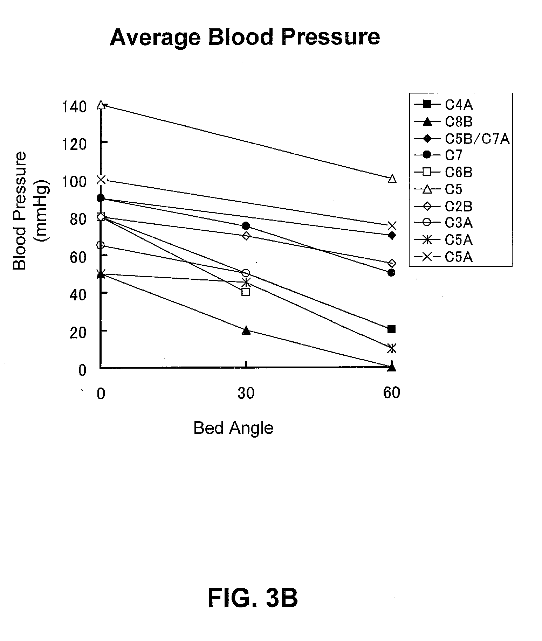

Figs. 3A and 3B are graphs showing the blood pressure change for patients with severe hypotension due to cervical and thoracic spinal cord injuries (10 patients with various injury levels in C4A-C5A). In both graphs, each patient in the supine position on a tilt table was raised to a 30 degree and to a 60 degree angle and his or her electrocardiogram and blood pressure were continuously recorded to measure the decrease in blood pressure. As a result, blood pressure decreases correlated to the bed inclination angle were observed for all of the patients.

The apparatus and method of the present embodiment prevent the orthostatic blood pressure decline in such patients with orthostatic hypotension due to spinal cord injury.

Electrode Positioning

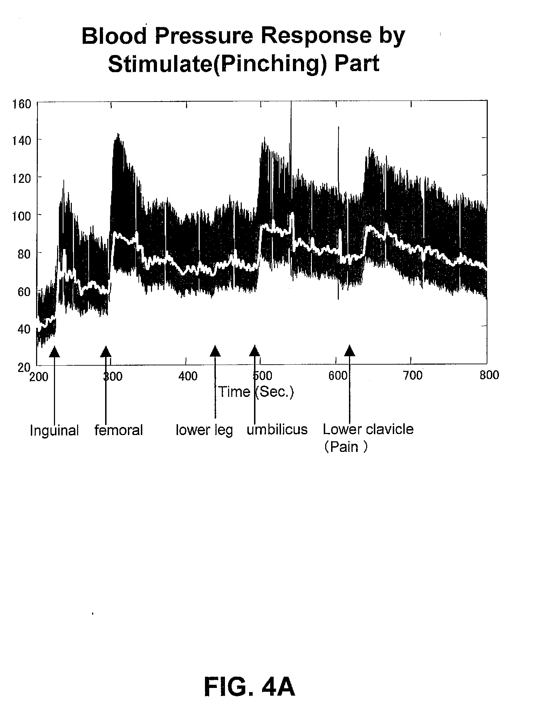

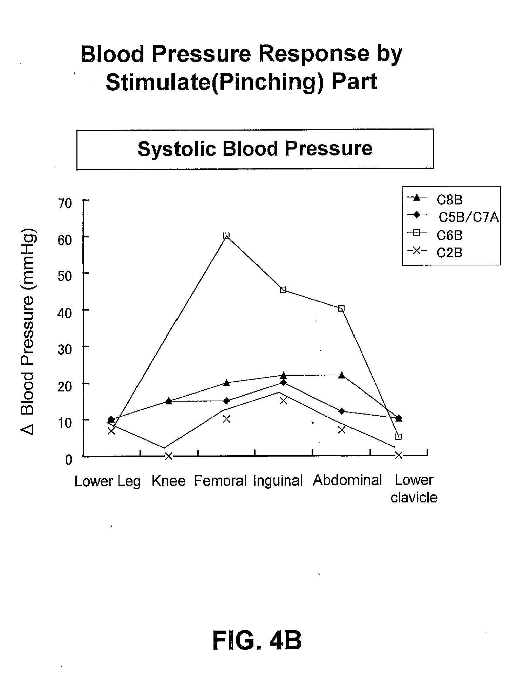

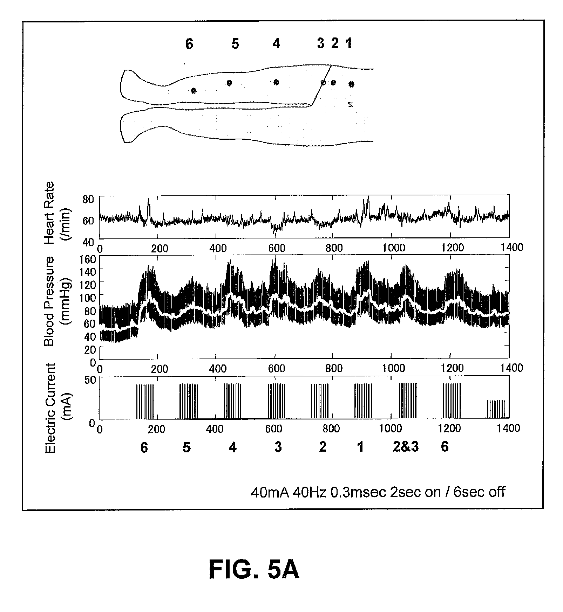

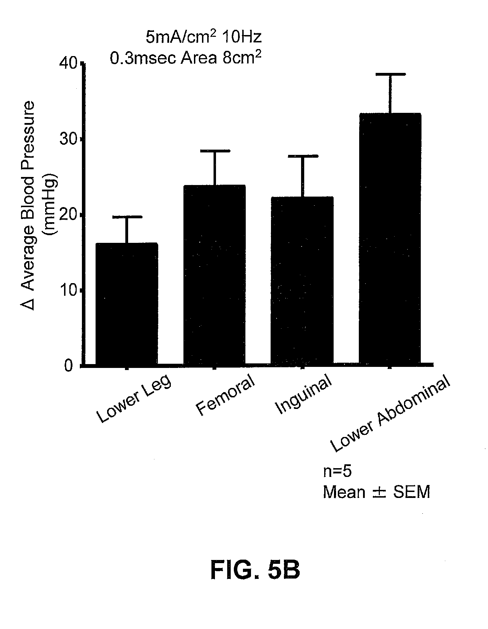

Figs. 4 and 5 are various graphs showing an optimal position to attach the electrode 4.

In this embodiment, in order to first identify the optimal attachment position, each of the lower leg, knee, femoral, inguinal, lower abdominal and subclavian regions of patients was stimulated by pinching, and the blood pressure increase due to the stimulus was observed, as shown in Figs. 4A and 4B. An electrode 4 was attached to each of the identified sites of the patient body and the elevation in systolic and average blood pressures were determined for the each site, resulting in relatively higher responses in the femoral, inguinal and lower abdominal regions, as shown in Figs. 5A and 5B.

As can be seen from these results, the optimal position to attach the electrode 4 is within the inguinal, femoral and lower abdominal regions although the electrode 4 may be attached to sites other than those identified as above depending on the patient body position or other reasons. Although the above results merely indicate the specific electrode attachment positions, the electrode may be attached to other sites such as lumbar region within the inguinal, femoral and lower abdominal regions. If the characteristics of the blood pressure increase response vary depending on the electrode attachment position, electrode characteristics may be adjusted accordingly in the set value storage section 9 and the electrode may be attached to the lower leg region or upper body as well.

Electric Current and Frequency Setting

The present apparatus may start its operation after the electrode 4 and blood pressure sensor 3 are attached to the patient skin and the power source 10 is connected. During its operation, when the patient blood pressure falls equal to or under the predetermined target value, it compensates the blood pressure decrease by applying an electric current with the predetermined frequency and amperage to the patient skin via the electrode 4.

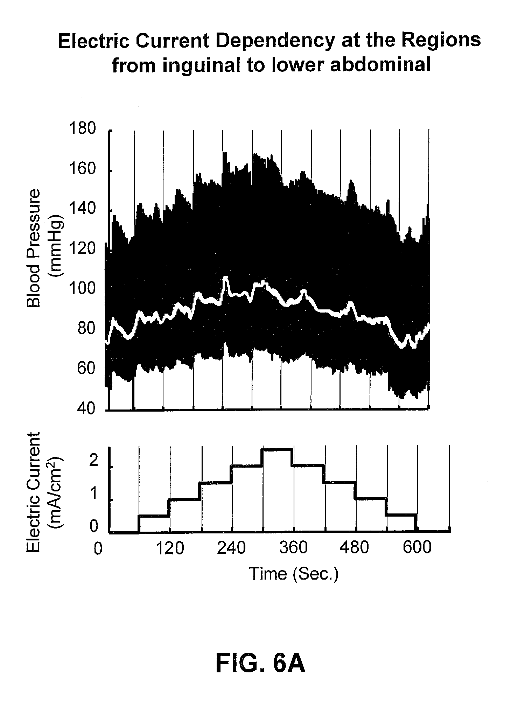

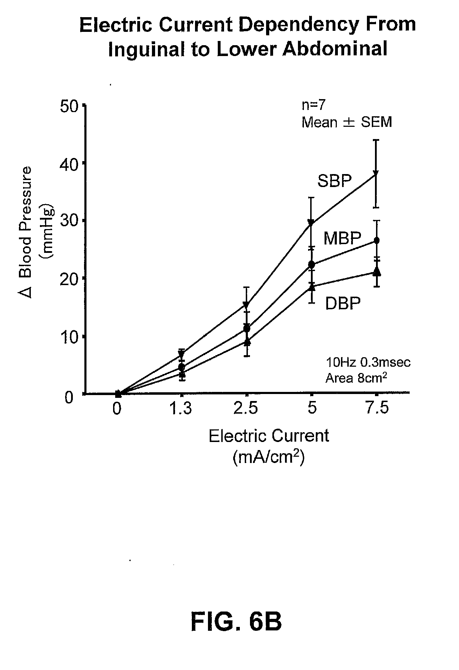

The results are shown below. The electrode 4 was attached to a desirable position within the inguinal and lower abdominal regions which had been identified to produce high responses in the above electrode positioning experiment. Figs. 6A and 6B are graphs showing the results of the blood pressure change obtained by attaching the electrode 4 to the desirable position within the inguinal and lower abdominal regions of the patient; and applying the electric current with a constant frequency at 10 Hz, square wave stimulations of 0.3 msec interval and varying amperage 0-7.5 mA/cm² with 0.5 mA/cm² steps, and elctrode area 8 cm². These graphs indicated that the patient blood pressure responded to the applied electric current and increased at any amperage and the responses positively correlated with the increase of the electric current. It should be noted that patients with spinal cord injury do not feel pain, and therefore it is possible and effective to raise the amperage of the applied electric current to an extent which does not damage the patient skin.

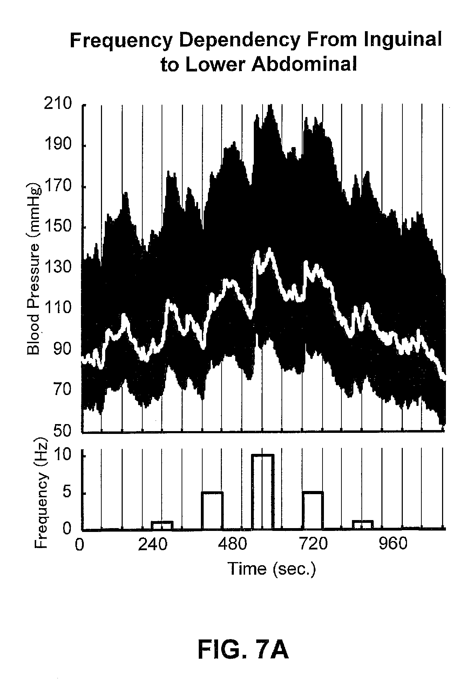

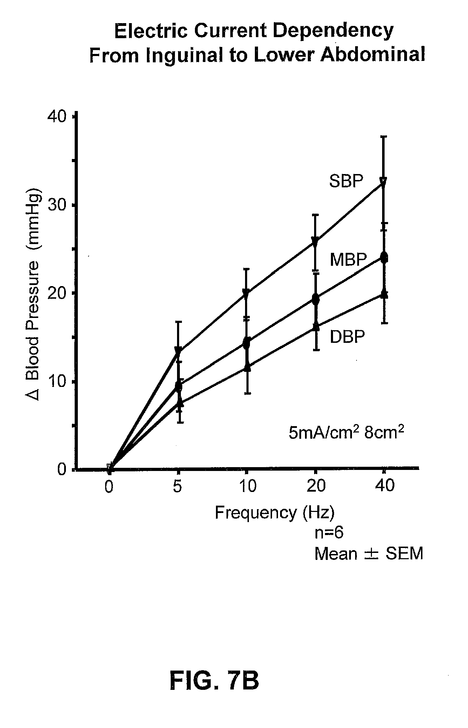

Figs. 7A and 7B are graphs showing the results of the blood pressure change obtained by attaching the electrode 4 to the desirable position within the inguinal and lower abdominal regions of the patient; and applying the electric current with a constant amperage 40 mA, square wave stimulations of 0.3 msec interval and different frequencies of 5 Hz, 10 Hz, 20 Hz and 40 Hz. These graphs indicate that the patient blood pressure responded to all of the frequencies of the applied electric current and the responses positively correlated with the frequency increase. At higher frequencies, however, patients experience discomfort due to the tonic muscle contraction and it is preferable to apply relatively lower frequencies and increase the electric current (amperage). For example, the muscle contraction discomfort caused at frequencies of about 10 Hz or higher is significant, but that caused below 10 Hz is considered small.

Electrode Size Setting

In this embodiment, the blood pressure change was examined in relation to different sizes of the electrode 4 in order to determine the size of the electrode 4 for optimal electric stimulation. As previously discussed, the electric stimulation was applied through the electrode attached to the inguinal region of the patients. As a result, the response increased with relatively smaller electrodes and with a plurality of electrodes rather than one. This suggests that the electric current density at the skin stimulation position governs the blood pressure response. This finding, in comparison with previous study reports, may be taken advantage of to achieve higher blood pressure responses with lower electric stimulation

According to this result, the optimal electrode size was determined to be 5 cm²-10 cm² in this embodiment, but sizes smaller or larger than this range may also be used, needless to say.

In addition, the number of the electrode is not limited to one and a plurality of electrodes may be used. In that case, the same or different sizes may be used for the plurality of electrodes. For example, when using two electrodes, they are preferably placed within the same region of the patient body at bilaterally symmetrical positions.

Intermittent Stimulation Conditions

As previously discussed, the present embodiment intermittently applies the electric current to the patient.

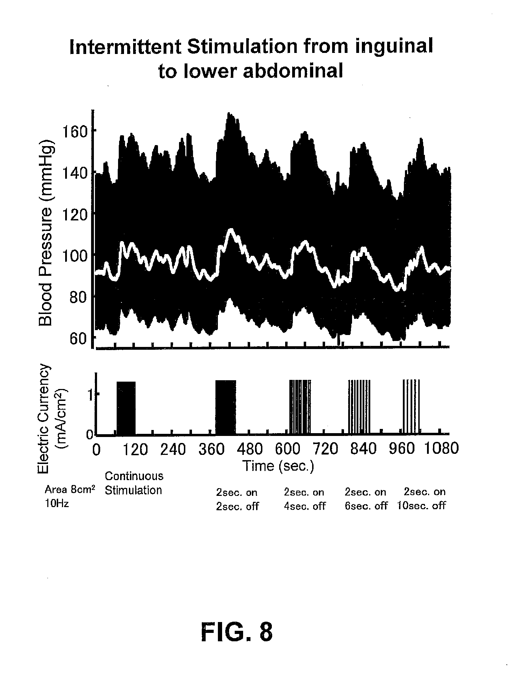

This intermittency is based on the fact that the blood pressure response declines to no effect in several minutes when the electric current stimulation is continuously applied to the patient skin. Fig. 8 is a graph showing the blood pressure increase response observed when the electrode 4 was attached to the desirable position within the patient's inguinal and lower abdominal regions and the electric current was applied with various intermittent time intervals (continuous; 2 sec. ON and 2 sec. OFF; 2 sec. ON and 4 sec. OFF; 2 sec. ON and 6 sec. OFF; and 2 sec. and 10 sec. OFF) in order to determine an appropriate time interval for the intermittent stimulation. As shown in Fig. 8, during the continuous stimulation, a tendency of gradual decrease in blood pressure was observed. It is effective to provide a non-stimulating interval (OFF period) longer than a stimulating interval, wherein an appropriate OFF period length needs to be evaluated since the blood pressure starts declining during prolonged non-stimulation. As optimal intermittent conditions, the stimulating interval is 1-3 sec. and non-stimulating interval 4-10 sec., and most preferably, the stimulating interval is 2 sec. and non-stimulating interval 6 sec.

Feedback Control

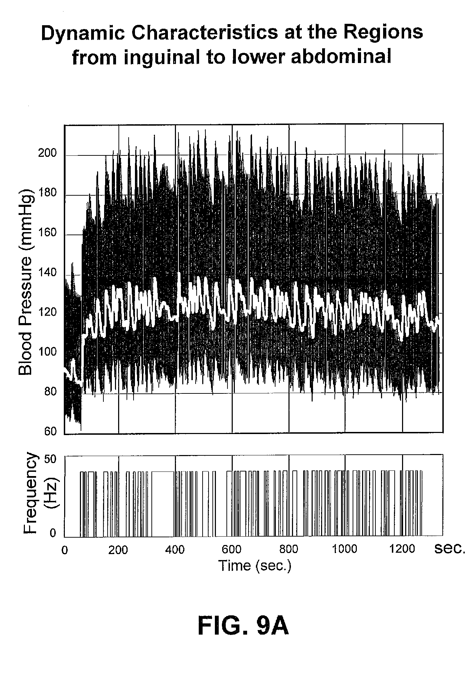

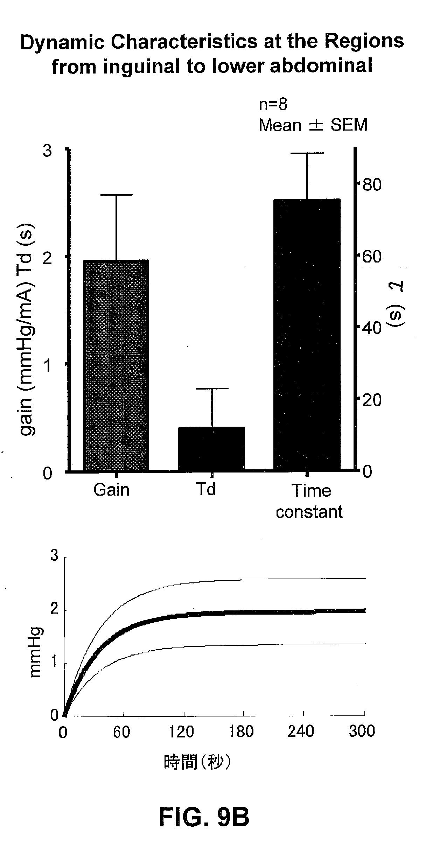

As discussed above, the control section prevents the patient blood pressure decrease and performs the feedback control to stabilize the blood pressure at the target value. In order to configure this feedback control, electric current-blood pressure dynamic characteristics were examined, as shown in Figs. 9A and 9B. From these results, generally common dynamic characteristics were found regardless of the severity of patient injury. These results indicate that the blood pressure decrease may be controlled by performing a certain electric control for any subject of treatment.

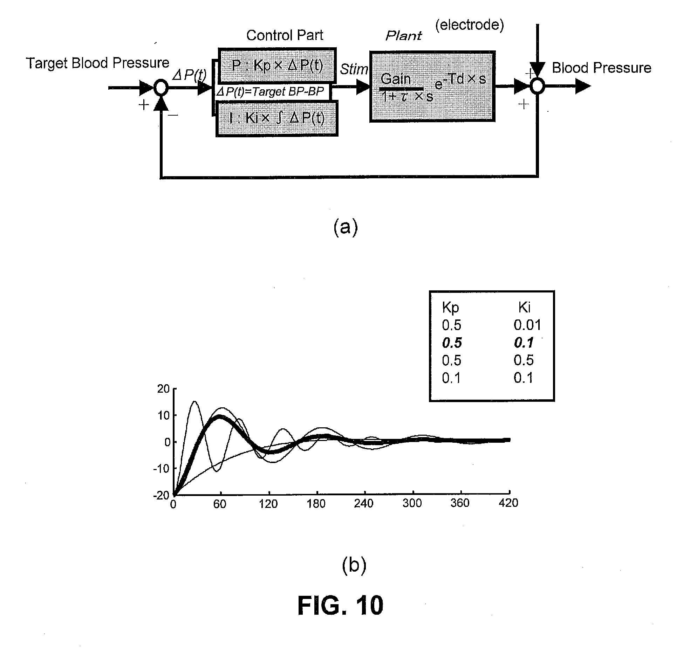

In order to estimate the transfer function and design a controller based on the above identified dynamic characteristics, the blood pressure dynamic response against the electric skin stimulation was identified using the white noise method or the step response, and an exemplary feedback-based controller was designed as shown in the block schematic diagram of Fig. 10A. Fig. 10B is a graph showing the response characteristics for different sets of the coefficients Kp and Ki. Although the example of Fig. 10 used the PID control scheme, the present invention is not limited to this scheme.

It should be noted that, when a plurality of electrodes are used, this control section may be provided depending on specific needs of the respective electrodes (electric current application sections), or a single control section may be control the plurality of electrodes.

Operational Examples

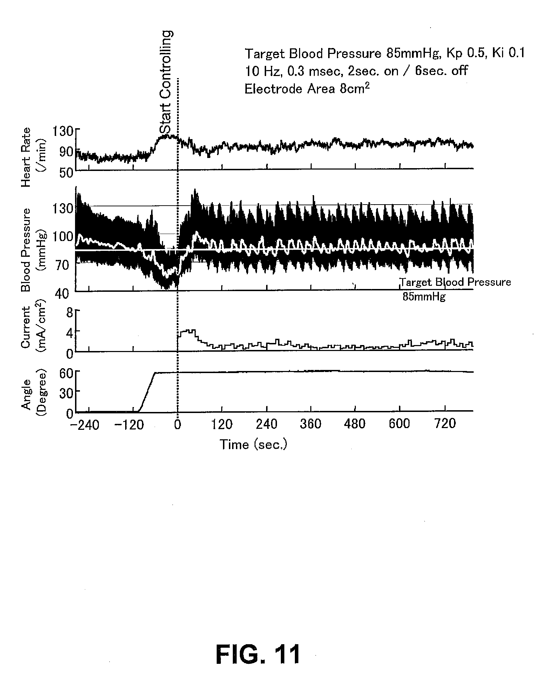

Fig. 11 shows the results of the blood pressure change in an actual study patient, wherein the varying electric stimulation was applied to the patient under the condition that the maximum amperage was 60 mA, target blood pressure value 85mmHg, frequency 10 Hz, stimulation interval 0.3 msec, 2 sec. on / 6 sec. off, and electrode area 8 cm². As evidenced by this figure, the blood pressure of the treated patient was maintained at the target blood pressure value of 85 mmHg by the intermittently applied electric stimulation.

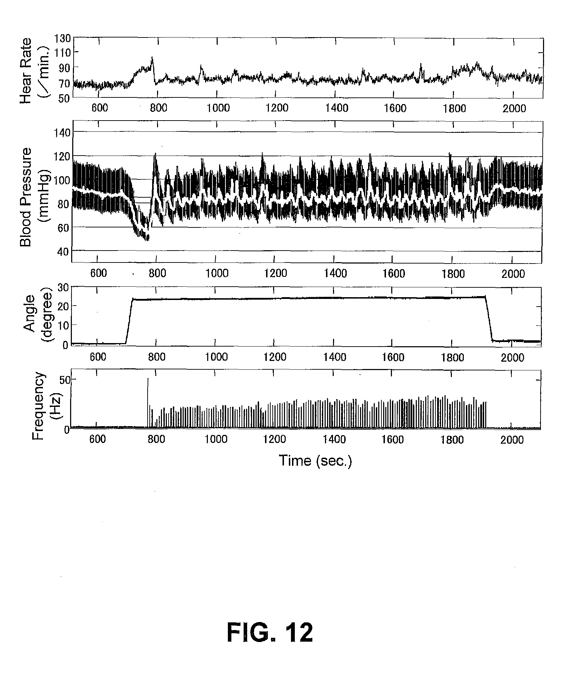

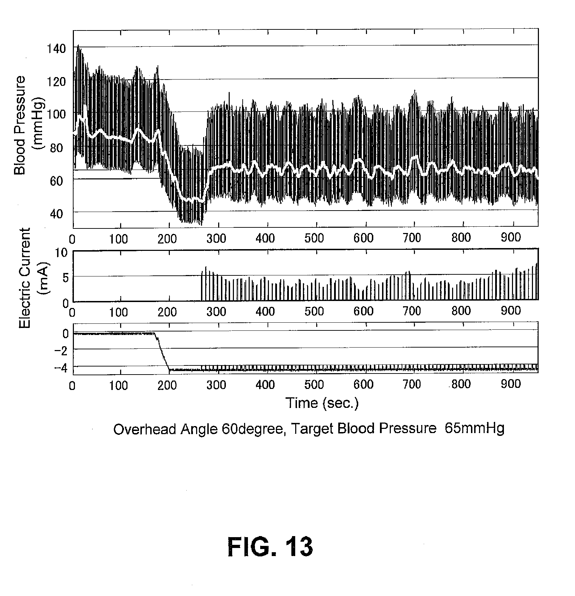

Also as another example of using the same apparatus, Fig. 12 shows the blood pressure change when the target blood pressure value was set to 85 mmHg, the frequency was kept constant and the electric current (amperage) was controlled. As a further example of using the same apparatus, Fig. 13 shows the blood pressure change when the target blood pressure value was set to 65 mmHg, the electric current (amperage) was kept constant and the frequency was controlled.

Thus, according to the apparatus of the present embodiment, the patient blood pressure may be stabilized at the target blood pressure value in a short period of time regardless of the severity of patient injury or other attributes by simply setting the target blood pressure value. In this embodiment, for example, the patient blood pressure can be increased to and maintained at the target blood pressure value within about 20 seconds.

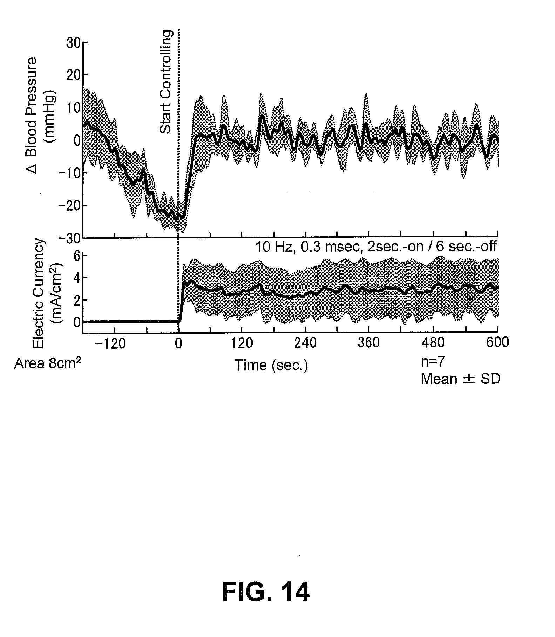

Moreover, Fig. 14 shows the average blood pressure change for 7 cases when the frequency was kept constant at 10 Hz, the maximum electric current (amperage) was set to 60 mA, and the current was controlled. In any of these cases, sustained blood pressure increase was observed with application of the intermittent electric stimulation and the blood pressure was found to be maintained near the target blood pressure value.

Apparatus Usage Example

Although the above described the case of patients in bed raised from the supine position, in the most typical usage example, the present apparatus may be secured to a wheelchair and attached to the patient in the wheelchair.

While the invention has been described, the present invention is not limited to the aforesaid examples and various changes and modifications can be made without departing from the spirit and scope of the invention.

For example, hypotension cases due to spinal cord injury have been discussed in the above embodiment, but the application of the present apparatus is not limited to hypotension and may also be applied to patients with systolic blood pressure lower than 100 mmHg.

In addition, the above one embodiment is provided with various components in the apparatus main body 2, but not limited to such a implementation and may be composed of a plurality of cases.

## Claims

1. An electric stimulation apparatus (1) for treating hypotension, **characterized by**:
a blood pressure measuring means (5) for continuously measuring a blood pressure of a subject;
an electric current application means (6) for intermittently applying an electric current with a predetermined frequency to skin of the subject to thereby stimulate the subj ect skin; and
a control means (7) for controlling the electric current application means (6) so as to maintain the blood pressure at a predetermined target blood pressure value by activating the electric current application means to stimulate the subject skin when the subject blood pressure is equal to or less than the target blood pressure value.

2. The electric stimulation apparatus (1) as in Claim 1, **characterized in that**
the electric current applied by the electric current application means (6) has an application period longer than a non-application period.

3. The electric stimulation apparatus (1) as in Claim 2, **characterized in that** the application period is 1-3 seconds and the non-application period is 4-10 seconds.

4. The electric stimulation apparatus (1) as in Claim 1, **characterized in that**
the electric current application means (6) comprises an electrode positioned within inguinal, femoral, lumbar and lower abdominal regions of the subject.

5. The electric stimulation apparatus (1) as in Claim 1, **characterized in that**
the control means (7) maintains the blood pressure of the subject at the target blood pressure value with a feedback control.

6. The electric stimulation apparatus (1) as in Claim 5, **characterized in that**
the control means (7) controls the applied electric current at a predetermined frequency.

7. The electric stimulation apparatus (1) as in Claim 5, **characterized in that**
the control means (7) controls the electric current application means (6) such that the applied electric current comprises a maximum current of 60 mA and a maximum frequency of 20 Hz.

8. The electric stimulation apparatus (1) as in Claim 1, further **characterized by**:
an operation means (7) for entering the target blood pressure value.

9. The electric stimulation apparatus (1) as in Claim 1, **characterized in that**
the electric stimulation apparatus is for treating hypotension due to spinal cord injury.

10. The electric stimulation apparatus (1) as in Claim 1, **characterized in that**
the electric stimulation apparatus is for treating orthostatic hypotension.

11. The electric stimulation apparatus (1) as in Claim 1, **characterized in that**
the electric stimulation apparatus is secured to a wheelchair.

12. An electric stimulation method for treating hypotension, **characterized by** the steps of:
continuously measuring a blood pressure of a subject;
intermittently applying an electric current with a predetermined frequency to skin of the subject to thereby stimulate the subject skin; and
controlling an electric current application means so as to maintain the blood pressure at a predetermined target blood pressure value by activating the electric current application means to stimulate the subject skin when the subject blood pressure is equal to or less than the target blood pressure value.

13. The electric stimulation method as in Claim 12, **characterized in that**
the electric current applied by the step of applying an electric current has an application period longer than a non-application period.

14. The electric stimulation method as in Claim 13, **characterized in that**
the application period is 1-3 seconds and the non-application period is 4-10 seconds.

15. The electric stimulation method as in Claim 12, **characterized in that**
an electrode is positioned within inguinal, femoral, lumbar and lower abdominal regions of the subject.

16. The electric stimulation method as in Claim 12, **characterized in that**
the step of controlling maintains the blood pressure of the subject at the target blood pressure value with a feedback control.

17. The electric stimulation method as in Claim 16, **characterized in that**
the step of controlling controls the applied electric current at a predetermined frequency.

18. The electric stimulation method as in Claim 16, **characterized in that**
the step of controlling controls the electric current application means such that the applied electric current comprises a maximum current of 60 mA and a maximum frequency of 20 Hz.

19. The electric stimulation method as in Claim 12, further **characterized by** the step of:
entering the target blood pressure value.

20. The electric stimulation method as in Claim 12, **characterized in that**
the electric stimulation method is for treating hypotension due to spinal cord injury.

21. The electric stimulation method as in Claim 12, **characterized in that**
the electric stimulation method is for treating orthostatic hypotension.
